# EUROPEAN PATENT SPECIFICATION

(11) **EP 2 978 411 B1**
(45) Date of publication and mention of the grant of the patent: **16.01.2019**
(21) Application number: 14727902.0
(22) Date of filing: 25.03.2014
(51) Int. Cl.: A61K 9/20

(54) **TABLET COMPRISING FRUCTOSE**
TABLETTE MIT FRUKTOSE
COMPRIMÉ COMPRENANT DU FRUCTOSE

(30) Priority: 25.03.2013 IT BO20130126
(43) Date of publication of application: 03.02.2016
(73) Proprietor: NATURALIA INGREDIENTS S.r.l, Mazara del Vallo (TP) (IT)
(72) Inventor: VALLINI, Veronica, I-60015 Falconara Marittima (IT); FORACI, Fabio, I-91026 Mazara Del Vallo (IT); GIOVANNONE, Daniele, I-03100 Frosinone (IT)
(74) Representative: Mangini, Simone
(86) International application number: PCT/IB2014/060125
(87) International publication number: WO 2014/155284

(56) References cited:
- WO-A1-90/14821
- CN-A- 101 829 066
- US-A1- 2011 048 413
- DRAY C ET AL: "Native fructose extracted from apple improves glucose tolerance in mice", JOURNAL OF PHYSIOLOGY AND BIOCHEMISTRY, vol. 65, no. 4, December 2009 (2009-12), pages 361-368, XP002717604, ISSN: 1138-7548 cited in the application

## Description

### TECHNICAL FIELD

The present invention concerns a tablet and a method for the production of a tablet.

### BACKGROUND TO THE INVENTION

Fructose is a simple natural carbohydrate, used in a wide range of food, dietary and pharmaceutical product formulations. It is advantageously used due to its high solubility (fructose in water is soluble up to approx. 80% at 20°C) and due to its sweetening properties. The sweetening profile of fructose is characterised both by a rapid perception of the sweet taste and an intensity which is the highest of the simple sugars (120% to 180% with respect to sucrose).

Due to its high sweetening power, fructose is the ideal sugar for applications in formulations with reduced calorie content, with obvious advantages in terms of finished product quality and flavour.

Furthermore, its very low glycemic index (approximately 20) makes fructose an ideal sweetener for the development of products with a low glycemic index, suitable for diabetics for example.

At commercial level crystalline fructose is produced mainly from high fructose corn syrups (HFCS) or from sucrose.

Direct compression is a technique frequently used for the production of tablets by the pharmaceutical and food industries. It is particularly advantageous with respect to other techniques as it produces compaction of the raw materials without recourse to further processes (mixing, drying, granulation) which often deteriorate the active ingredients carried, which are easily degradable. Direct compression requires a new and critical approach in terms of the selection of raw materials and excipients, the flow properties of the mixtures and the effects of formulation variables on compressibility.

The direct compression of materials and/or active ingredients offers many advantages:
- ECONOMY: savings are obtained from reduction of the process times, the number of steps involved, and the equipment required;
- DISINTEGRATION OF THE TABLET INTO PRIMARY PARTICLES OF ACTIVE INGREDIENT: the obtaining of fine particles and not granules from disintegration of the tablets constitutes an undeniable advantage for the bioavailability of the active ingredient since the increase in the contact surface with the biological fluids makes solubilisation thereof more rapid;
- ELIMINATION OF HUMIDITY AND HEAT and consequent STABILITY: this is the most significant advantage in terms of quality of the tablet. The humidity and heat inherent in the wet granulation process and the high compaction pressures required are generally harmful to the active ingredients present in the formulation.

A suitable material for direct compression should have the following rheological properties:
- Free-flowing: to uniformly fill the compression machine mould;
- Lubricating capacity: necessary to ensure that the material subject to compression does not stick to the punches used during compression;
- Compressibility: related to its binding power without having to apply high compression forces which could negatively affect the active substances present in the formulation;
- Disintegrant capacity: so that the tablet formed has an acceptable disintegration time in water or in the organic matrices,

Many ingredients are not suitable for direct compression due to their insufficient flowability and/or compressibility.

In the formulation of tablets, sugars are suitable for use as a raw material due to their organoleptic properties. However, the majority of crystalline sugars, in particular fructose, are not particularly suitable for direct compression, due to their poor flowability and compressibility. This is due mainly to the nature of the pure anhydrous fructose in crystalline form, the typical configuration of which makes it unsuitable for direct compression in a compression machine. Document WO 90/14821 A1 discloses the preparation of tablets comprising granulated fructose.

To remedy this problem granular formulations of sugars have been proposed, suitable to be used for direct compression. Examples of commercial products are: EMDEX (dextrose agglomerate); DIPAC (sucrose agglomerate containing dextrins); STARCH 1500 (pregelatinized starch and mannitol).

Object of the present invention is to provide a tablet and a method for the production of a tablet which overcome, at least partially, the drawbacks of the known art and, at the same time, are easy and inexpensive to produce.

### SUMMARY

According to the present invention a tablet and a method for the production of a tablet are provided according to the independent claims that follow and, preferably, any one of the claims depending directly or indirectly on the independent claims.

### BRIEF DESCRIPTION OF THE FIGURES

- figures 1 to 3 are photographs of tablets not according to the present invention;
- figures 4 to 6 are photographs of tablets according to the present invention;
- figures 7 to 9 are SEM photographs of fructose crystals obtained from grapes; and
- figures 10 to 12 are SEM photographs of fructose crystals of different origin.

### EMBODIMENTS OF THE INVENTION

According to a first aspect of the present invention, a solid tablet is provided comprising at least 60% by weight, with respect to the total weight of the tablet, of fructose derived (extracted) from fruit. More precisely, the above-mentioned fructose is derived (in particular, extracted) from grapes wherein the above-mentioned fructose is crystalline. The crystallinity may be verified, for example, by normal crystallography techniques (for example X-ray diffraction, of neutrons and/or electrons).

Advantageously, the tablet comprises at least 70% (in particular, at least 90%) by weight, with respect to the total weight of the tablet, of fructose derived from fruit. According to particular embodiments, the tablet comprises at least 95% (in particular, at least 97%) by weight, with respect to the total weight of the tablet, of fructose derived from fruit.

In particular, the tablet comprises up to 99% by weight, with respect to the total weight of the tablet, of fructose derived from fruit. According to some embodiments, the tablet consists (entirely) of fructose derived from fruit.

It has been experimentally observed that, unlike pure crystalline fructose of different origin, fructose derived from fruit (in particular, grapes) surprisingly has excellent characteristics of compressibility, also at very high concentrations, up to 100%.

The tablet according to the present invention, in particular, has a relatively low tendency to change colour and to cracking off, it is not very friable and is much more resistant to the stability tests. On the other hand, tablets obtained with crystalline fructose with origin different from fruit have shown very different and unsatisfactory characteristics.

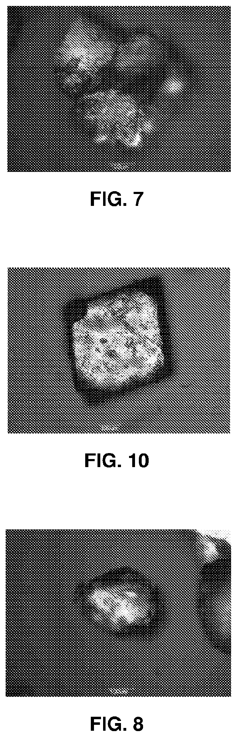

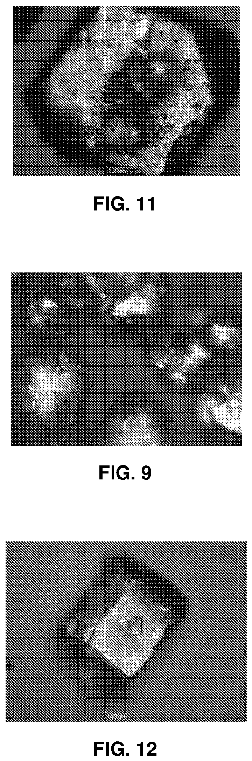

It may be assumed that the greater compressibility of the fructose derived from fruit is for the most part due to a different crystalline structure. In fact, the fructose crystals from grapes appear more irregular under microscopic analysis (see figures 7 to 12).

It should be noted that the substantial difference between the fructose derived from fruit and the fructose of other origins (for example from maize, sugar cane and/or beet etc.) is confirmed by some studies on mice (of which we cite Dray et al., 2009 "Native fructose extracted from apple improves glucose tolerance in mice" Journal of Physiology and Biochemistry), followed by clinical trials on humans. These tests have shown that the fructose extracted from fruit has an extremely low glycemic index (12 vs. 20 characteristic of the "traditional" maize fructose) and results in very low insulin secretion.

The tablets according to the present invention may be used as sweeteners with reduced calorie content and low glycemic impact, or as energizers (the fructose represents a slow-release energy source which may cover protracted physical exertion).

It is important to point out that the origin of the fructose (from fruit or other) may be identified by means of known techniques. In particular, the fructose may be converted by means of appropriate fermentation into ethyl alcohol, which may be subjected to analyses for identifying the isotope ratios (more precisely, the deuterium/hydrogen - D/H - ratios and the ratios between the carbon isotopes ¹³C/¹²C). The methods applied are OIV-MA-AS311-05 (SNIF-NMR) and OIV-MA-AS312-06 (EA-IRMS). See also Claudia Bauer-Cristoph et al. "Assignment of raw material and authentication of spirits by gas chromatography, hydrogen- and carbon-isotope ratio measurements"; Z Lebensm Unters Forsch A (1997) 204: 445-452.

In particular, it is possible to use SNIF-NMR (specific natural isotope fractionation studied by nuclear magnetic resonance - to determine the distribution of the deuterium in the ethanol) and IRMS (isotope ratio mass spectrometry - to determine the isotope ratio ¹³C/¹²C) to confirm (or not) the origin of the fructose derived from fruit (in particular, from grapes). More specifically, the SNIF-NMR analysis identifies the presence of beet sugar while the IRMS analysis identifies the presence of cane sugar/maize or other plant with C4 photosynthetic cycle.

These valuations are based on the following principles.

The deuterium contained in the sugars following fermentation is redistributed in the molecules I (CH₂DCH₂OH), II (CH₃CHDOH), III (CH₃CH₂OD) and IV (HOD).

Considering (D/H)I = isotope ratio associated with molecule I and (D/H)II = isotope ratio associated with molecule II, it follows that R (R = 2 (D/H)II/(D/H)I) expresses the relative distribution of the deuterium in the molecules I and II. R is measured directly on the basis of the intensities h of the signals and, therefore, R = 3hII /hI.

The determination of the parameters just defined as R, (D/H)I and (D/H)II is performed by NMR of the deuterium on the ethanol extracted after fermentation.

During the photosynthesis, the carbon dioxide is assimilated by the plants via two main types of metabolism: C3 metabolism (Calvin cycle) and C4 metabolism (Hatch and Slack). These two photosynthesis mechanisms have a different isotope fractionation. The products derived from C4 plants, like the sugars and the alcohol derived by fermentation, therefore have higher levels of carbon-13 than the corresponding products derived from C3 plants. The majority of plants, including vines and sugar beet, belong to the C3 group. Sugar cane and maize belong to the C4 group. Measurement of the carbon-13 level therefore allows identification and quantification of the sugar of C4 origin (cane sugar or maize isoglucose).

The information on the level of carbon-13 combined with the information obtained by NMR-SNIF also allow quantification of the addition of mixtures of original sugars or alcohols of the C3 and C4 plants.

Advantageously, the fructose of the solid tablet may be identified as derived from fruit (in particular, from grapes) using the methods OIV-MA-AS311-05 (SNIF-NMR) and OIV-MA-AS312-06 (EA-IRMS).

These methods may be crossed if necessary with other known analytical techniques to identify the origin of the fructose contained in the tablets.

It should be noted, among other things, that in the sugars derived from fruit (in particular, from grapes) and, specifically, in the fructose, inositol is present (in particular, in traces) contrarily to what occurs with fructose of other origin.

In view of the above, typically, the fructose (of the tablet) derived from fruit (in particular, from grapes) has a δ¹³C(‰)vs. V-PDB ranging from -24 to -30.

It should be noted that δ¹³C represents the content of Carbon-13 expressed in parts per 100C (‰) and V-PDB (Vienna-Pee-Dee Belemnite) is the main reference for measurement of the natural variations in the isotope contents of Carbon-13.

In addition or alternatively, the fructose (of the tablet) derived from fruit (in particular, from grapes) has an R value higher than 2.4 and lower than 2.6. In addition or alternatively, the grape fructose comprises (traces of) inositol.

In addition or alternatively, the fructose (of the tablet) derived from fruit (in particular from grapes) has a (D/H)I value from 99 to 105.

According to some embodiments, the tablet also comprises a disintegrant (which may also be a combination of several disintegrants). More precisely, the tablet comprises (from 0.5%) up to 5% by weight of a disintegrant, with respect to the total weight of the tablet. In other words, the tablet does not comprise more than 5% by weight of disintegrant (or of the sum of different disintegrants).

In some cases, the tablet is substantially without disintegrants.

In particular, the disintegrant is selected from the group consisting of: polyvinylpyrrolidone, polyvinylpolypyrrolidone, starch, starch derivatives, carboxymethyl cellulose, salts (and derivatives) of carboxymethyl cellulose, precipitated silica (and a combination thereof). More precisely, the disintegrant is selected from the group consisting of: polyvinylpyrrolidone, starch (and a combination thereof),

It has been experimentally observed that these tablets, in addition to being able to dissolve more easily in water, surprisingly have a particularly high stability to humidity.

According to some embodiments, the tablet also comprises one or more binders. In particular, the tablet comprises a percentage by weight of one or more binders (from 0.5%) up to 10% (in particular, lower than 5%) by weight, with respect to the total weight of the tablet. In other words, the tablet does not comprise more than 10% of the binder (or of the sum of different binders).

In some cases, the tablet is substantially without binders.

In particular, the one or more binders are selected from the group consisting of: dextrose, sucrose, dextrin, pregelatinized starch, mannitol (and a combination thereof).

According to some embodiments, the tablet also comprises an anti-adherent lubricant (which may also be a combination of several anti-adherent lubricants). In particular, the tablet comprises (from 0.5%) up to 5% in weight, with respect to the total weight of the tablet, of the anti-adherent lubricant. In other words, the tablet does not comprise more than 5% by weight of the anti-adherent lubricant (or of the sum of different anti-adherent lubricants).

In some cases, the tablet is substantially without anti-adherent lubricants.

In particular, the anti-adherent lubricant is selected from the group consisting of: starch, silica, stearates, talc, wax, polyethylene glycol, sodium/magnesium lauryl sulphate, leucine (and a combination thereof).

It should be noted that if a single compound has a dual function, its concentration will be lower than or equal to the maximum scheduled for one of the two types of components. For example, if a single component acts both as a disintegrant and lubricant, its concentration in the tablet will be up to 5% by weight.

According to some embodiments, the tablet comprises a functional component (which may also be a combination of several functional components). In particular, the tablet comprises (from 0.5%) up to 40% (more precisely, up to 30%) by weight, with respect to the total weight of the tablet, of the functional component. In other words, the tablet does not comprise more than 30% by weight of the functional component (or of the sum of different functional components).

In particular, the functional component is selected from the group consisting of: vitamins, amino acids, mineral salts, antioxidants (more precisely, grape polyphenols), plant extracts (and a combination thereof).

In some cases, the tablet is substantially without functional components.

According to some embodiments, the tablet comprises a high-intensity sweetener (which may also be a combination of several high-intensity sweeteners). In particular, the tablet comprises (from 0.5%) up to 30% (more precisely, up to 25%), with respect to the total weight of the tablet, of the high-intensity sweetener. In other words, the tablet does not comprise more than 30% (in particular, no more than 25%) by weight of the high-intensity sweetener (or of the sum of different high-intensity sweeteners).

In some cases, the tablet is substantially without high-intensity sweeteners.

The high-intensity sweeteners are substances with a high sweetening power, usually from approximately 30 to 10,000 times that of sucrose. Since very small quantities are sufficient to obtain the desired sweetening effect, their calorific power is very low and practically null.

Some of the most widespread high-intensity sweeteners are: acesulfame K, aspartame, cyclamate, neohesperidin DC, neotame, saccharin, sucralose, thaumatin, steviol glycosides (Stevioside, Rebaudioside A, B, C, Dulcoside), relative salts (and a combination thereof).

Advantageously, the fructose derived from fruit has a grain size from 50 µm (in particular, from 200 µm) to 600 µm (in particular, to 400 µm).

The grain size of fructose is measured by the ICUMSA GS2-37 method.

According to some embodiments, the tablet has a weight from 0.05 grams to 10 grams.

According to some embodiments, the tablet is obtainable (obtained) in accordance with the method (reported below) as per the second aspect of the present invention.

In accordance with a second aspect of the present invention, a method is provided for the production of a tablet comprising at least 60% by weight, with respect to the total weight of the tablet, of fructose derived from fruit (in particular from grapes). The method comprises a compression step, during which a formulation, comprising at least 60% by weight, with respect to the total weight of the formulation, of the fructose derived from fruit (in particular from grapes), is processed by means of direct compression to obtain the tablet.

In particular, the method comprises a production step, during which the fructose is obtained (extracted) from the fruit (in particular, from grapes).

More precisely, the production step is performed according to EP1734108, US7935189 and PCTIB2012054210. In these documents a method is described for extracting fructose from grape juice and crystallising it by cooling, without the use of additional solvents. The crystalline fructose obtained has a purity higher than 98%, grain size varying from 0.2 to 0.6 mm and complies with the requirements of the Directive 2011/111/EC, the Food *Chemical Codex* and the *Codex Alimentarius.*

In particular, the fructose is as defined in accordance with the first aspect of the present invention.

According to some embodiments, the tablet is as defined in accordance with the first aspect of the present invention.

Advantageously, the mixture comprises a binder in accordance with the indications relative to the tablet described in the first aspect of the present invention. Furthermore, the percentage content by weight of the binder in the mixture is as indicated with regard to the tablet.

In particular, in some cases, the mixture is without binder.

According to some embodiments, the mixture comprises a disintegrant according to the indications relative to the tablet in the first aspect of the invention. Furthermore, the percentage content by weight of the disintegrant in the mixture is as indicated with regard to the tablet.

In particular, in some cases, the mixture is without the disintegrant.
According to some embodiments, the mixture comprises an anti-adherent lubricant according to the indications relative to the tablet described in the first aspect of the present invention. Furthermore, the percentage content by weight of the anti-adherent lubricant in the mixture is as indicated with regard to the tablet.
In particular, in some cases, the mixture is without the anti-adherent lubricant.
According to some embodiments, the mixture comprises a high-intensity sweetener in accordance with the indications relative to the tablet described in the first aspect of the present invention. Furthermore, the percentage content by weight of the high-intensity sweetener in the mixture is as indicated with regard to the tablet.
According to some embodiments, the mixture comprises a functional component according to the indications relative to the tablet described in the first aspect of the present invention. Furthermore, the percentage content by weight of the functional component in the mixture is as indicated with regard to the tablet.

### Example 1

### Comparison between "traditional" commercial fructose and grape fructose in direct compression

Three different industrial batches of crystalline grape fructose (Naturalia Ingredients) and three different commercial samples of fructose of different origin (cereals and/or sucrose) indicated as suitable for use in compression by the suppliers (Galam, Tate & Tyle) were tested in direct compression.

The formulation of the tablets was the same: 98% fructose + 2% processing aid (machine lubricant - leucine (KYOWA®)).

The tablets were prepared by direct compression, after 15 min. mixing of the ingredients, using a Korsch XL 400 compression machine with force of 12kN and rotation speed of 20,000 tablets/h.

The three batches of grape fructose showed similar repeatable characteristics. The three samples of commercial fructose used as controls showed very different characteristics (not particularly suitable for compression, one due to colour, one due to friability and one due to machinability). In direct compression the grape fructose compressed better, did not change colour and was more stable.

Tables 1 and 2 show the characteristics of the tablets obtained with the three samples of commercial fructose and the three samples of grape fructose respectively.

**Table 1**

| | **Commercial fructose** | | |
|---|---|---|---|
| | **C1** | **C2** | **C3** |
| Mean grain size | 0.6 | 0.4 | 0.2 |
| Tablet weight | 2.5 g | 2.76 g | 2.43 g |
| Hardness | 10 KP | 5.1 KP | 2.6 KP |
| Thickness | 5.2 mm | 5.5 mm | 5 mm |
| Disintegration | 8 minutes | 7 minutes | 5 minutes |
| Flowability | good | good | low |
| Compressibility | very poor | very poor | very poor |
| Adhesion | YES | no | no |
| Cracking off | YES | YES | YES |
| Friability | YES | YES | YES |
| **Tablet result** | VERY POOR | Friable | VERY POOR |

**Table 2**

| | **Grape fructose** | | |
|---|---|---|---|
| | **U1** | **U2** | **U3** |
| Mean grain size | 0.3 | 0.4 | 0.5 |
| Tablet weight | 2.5 g | 2.55 g | 2.52 g |
| Hardness | 12 KP | 6.4 KP | 5.9 KP |
| Thickness | 5.1 mm | 5 mm | 5 mm |
| Disintegration | 8 minutes | 5 minutes | 6 minutes |
| Flowability | good | good | good |
| Compressibility | fair | good | good |
| Adhesion | low | no | no |
| Cracking off | null | null | null |
| Friability | null | null | null |
| **Tablet result** | GOOD | GOOD | GOOD |

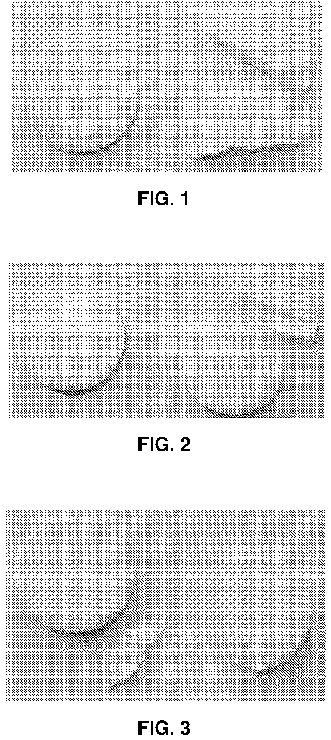

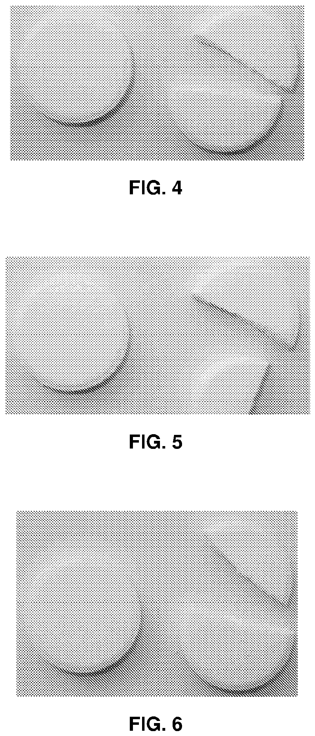

Figures 1 to 3 are photographs of tablets obtained with the commercial fructose C1-C3 respectively. Figures 4 to 6 are photographs of tablets obtained with the grape fructose U1-U3 respectively.

The measurements of the technological characteristics reported in tables 1 and 2 were performed as below.

Grain size: the grain size was measured by means of the ICUMSA method (GS2-37).

Tablet weight: the mean weight of the tablets was determined by individually weighing 20 tablets using an analytical balance (European Pharmacopoeia).

Hardness: the hardness was determined as a mean of 10 tablets using a Sotax HT 10 durometer.

Thickness: the thickness was determined using an appropriately calibrated and validated gauge.

Disintegration time: the disintegration time was measured as established by the European Pharmacopoeia (current edition).

Flowability: the flowability was evaluated by measuring the helical angle of the granulate with the FT4 Powder Rheometer (note: in our specific case an evaluation by the operator was performed, not a measurement).

Compressibility: the compressibility was evaluated by measuring the compression force of the compression machine used and the hardness of the tablet. With the same hardness, the formulation that requires less compression force is more compressible.

Adhesion: the adhesion was evaluated directly by the operator by observing the appearance of the tablet and the glossiness of the punches of the compression machine used. A glossy tablet with glossy punches is synonymous with an original formulation having excellent anti-adherent properties.

Cracking off: the cracking off was evaluated directly by the machine operator by observing the tablets both during mould ejection and during the friability test. The tablets must never crack into two mirror-image parts with crack horizontal to the edge, or have visible cracks on the same edge. Friability: friability is measured with the friabilimeter and relative method described in Pharmacopoeia Europea (note: in our specific case, an evaluation by the operator was performed, not a measurement).

As may be easily seen from the data and figures 1-6, the tablets obtained from grape fructose surprisingly proved to be of considerably superior quality. In particular, as regards the commercial fructose, the compressibility is very poor, and the tablets are altered in terms of colour (tendency to form yellow patches) and/or friability and/or with tendency to cracking off. The crystalline grape fructose, on the other hand, at the same concentrations and operating conditions, compresses well and the product obtained does not change colour, is not friable, does not tend to cracking off and is more resistant to the stability tests.

### Example 2

### Grape fructose based tablets

Following the methodology described in the previous example, tablets were prepared with the following compositions.

**Table 3**

| **Composition** | **Grape A** | **Grape B** |
|---|---|---|
| Grape fructose | 94% | 94 % |
| Disintegrant | 3% (INF-10) | 3% (dried starch) |
| Machine lubricant (leucine) | 3% | 3% |

INF-10 is a polyvinylpyrrolidone with trade name Polyplasdone® INF-10 ISP (ISP International speciality products). The starch was supplied by Egman Veronelli, Prodotti Gianni.

These tablets may be used as sweeteners or as energizers (fructose is a source of slow release energy).

The tablets obtained showed the following technological characteristics (measured as described in the previous example).

**Table 4**

| **Characteristics** | **Grape A** | **Grape B** |
|---|---|---|
| Tablet weight | 2.47 g | 2.51 |
| Hardness | 6.2 KP | 5.6 KP |
| Thickness | 5.3 mm | 5.3 mm |
| Disintegration time | 2 minutes | approx. 30 seconds |
| Flowability | good | good |
| Compressibility | good | good |
| Adhesion | no | no |
| Cracking off | no | no |
| Friability | no | no |
| NOTES | Good | Good |

Compared to the tablets described in example 1, these tablets are more disintegrable and surprisingly more stable to humidity.

### Example 3

### Grape fructose based tablets

Following the methodology described in example 1 tablets may be produced also on the basis of the following formulations, in which the disintegrant and the lubricant are as described in the previous example.

**Table 5**

| **Composition A** | **Grams per tablet (2g)** | **% weight** |
|---|---|---|
| Grape fructose | 1. 87 | 93.5% |
| Disintegrant (starch) | 0.06 | 3% |
| Machine lubricant (leucine) | 0.06 | 3% |
| Steviol glycosides (95%) | 0.01 | 0.5% |

The product RA95 is used (supplier: Cargill®) as a steviol glycoside.

These tablets (composition A) may be used as sweeteners with reduced calorie content and very low glycemic impact. A 2 g tablet sweetens like one dose (= 6 grams of sucrose), but contributes 7.5 kcal as against 24 of the same dose of sucrose, providing a calorie saving of approximately 70%, in addition to a very low glycemic impact.

**Table 6**

| **Composition B** | **Grams per tablet (2g)** | **% weight** |
|---|---|---|
| Grape fructose | 1.7 | 85 |
| Grape polyphenols (e.g. opc) | 0.3 | 15 |

Grape polyphenols may be obtained by extraction from grapes. These tablets (composition B) may be used as dietary supplements with antioxidant action.

**Table 7**

| **Composition C** | **Grams per tablet (5g)** | **% weight** |
|---|---|---|
| Grape fructose | 3.5 | 70 |
| Vitamin complex* | 0.02 | 0.4 |
| Energizer mixture** | 1.48 | 29.6 |

| | | |
|---|---|---|
| * Riboflavin, Niacin, Vitamin B6, Vitamin B12, pantothenic acid, folates (supplier: Roche). ** taurine, d-gluconolactone, caffeine, Ginkgo Biloba (supplier Polichimica). | | |

These tablets (composition C) may be used as dietary supplements with energizing action, combining the slow release energy action of the fructose with the functions of the specific active ingredients.

**Table 8**

| **Composition D** | **Grams per tablet (2g)** | **% weight** |
|---|---|---|
| Grape fructose | 1.5 | 75 |
| N-acetyl carnitine | 0.5 | 25 |

These tablets (composition D) may be used as dietary supplements with antiasthenic action, combining the slow release energy action of the fructose with the functions of the specific active ingredients.

## Claims

1. A tablet comprising at least 60% by weight, with respect to the total weight of the tablet, of fructose derived from fruit, in particular from grapes; wherein fructose is crystalline and has a δ¹³C(‰)vs V-PDB ranging from -30 and -24 and a R value higher than 2.4 and lower than 2.6.

2. A tablet according to claim 1 and comprising at least 70%, in particular at least 90%, by weight, with respect to the total weight of the tablet, of fructose derived from fruit.

3. A tablet according to one of the previous claims and comprising at least one disintegrant; the tablet has up to 5% by weight, with respect to the total weight of the tablet, of said disintegrant; in particular, said disintegrant is selected in the group consisting of: polyvinylpyrrolidone, polyvinylpolypyrrolidone, starch, starch derivatives, carboxymethyl cellulose, salts and derivatives of carboxymethyl cellulose and a combination thereof.

4. A tablet according to one of the previous claims and having a percentage by weight of one or more binders that is lower than 10%, with respect to the total weight of the tablet; in particular, said one or more binders are selected in the group consisting of: dextrose, sucrose, dextrin, pregelatinized starch, mannitol and a combination thereof.

5. A tablet according to claim 4, wherein the tablet is without binders.

6. A tablet according to any of the previous claims and comprising up to 5% by weight, with respect to the total weight of the tablet, of at least one anti-adherent lubricant; in particular, said anti-adherent lubricant is selected in the group consisting of: starch, silica, stearates, talc, wax, polyethylene glycol, sodium/magnesium lauril sulfate, leucine and a combination thereof.

7. A tablet according to any of the previous claims and comprising at least one high-intensity sweetener; in particular, the tablet comprises up to 30% by weight, with respect to the total weight of the tablet, of said high-intensity sweetener.

8. A tablet according to any of the previous claims and comprising a functional component; in particular, the tablet comprises up to 40% by weight, with respect to the total weight of the tablet, of said functional component; in particular, said functional component is selected in the group consisting of: vitamins, amino acids, dietary minerals, antioxidants, plant extracts and a combination thereof.

9. A tablet according to any of the previous claims and having a weight ranging from 0.05 grams to 10 grams.

10. A method for manufacturing a tablet comprising at least 60% by weight, with respect to the total weight of the tablet, of fructose derived from fruit, in particular from grapes;
wherein the tablet is according to any one of the claims from 1 to 9;
said method
comprises:
a compression step, during which a formulation comprising at least 60% by weight, with respect to the total weight of the formulation, of fructose derived from fruit is processed by means of compression so as to obtain the tablet.

11. A method according to claim 10, and comprising a mixing step, during which the fructose derived from fruit (in particular from grapes) is mixed so as to obtain said formulation; the formulation having a percentage by weight of one or more binders that is lower than 10%, with respect to the total weight of the tablet; in particular, said one or more binders are selected in the group consisting of: dextrose, sucrose, dextrin, pregelatinized starch, mannitol and a combination thereof.

12. A method according to claim 10 or 11, wherein the mixture is without binders.

13. A method according to any of the claims from 10 to 12, wherein the fructose derived from fruit has a grain size ranging from 50 µm to 600 µm.

14. A tablet according to any of the claims from 1 to 9 and obtainable by the method according to any one of the claims from 10 to 13.

## Patentansprüche

1. Tablette, umfassend, bezogen auf das Gesamtgewicht der Tablette mindestens 60 Gew.-% an von Früchten, insbesondere von Trauben stammender Fruktose; wobei Fructose kristallin ist und ein δ¹³C (‰) vs V-PDB im Bereich von -30 und -24 und einen R-Wert von mehr als 2.4 und weniger als 2.6 aufweist.

2. Tablette nach Anspruch 1, umfassend, bezogen auf das Gesamtgewicht der Tablette mindestens 70 Gew.-%, insbesondere mindestens 90 Gew.-%, an von aus Fruchtgewonnener oder an von Früchten stammender Fruktose.

3. Tablette nach einem der vorhergehenden Ansprüche und umfassend mindestens einen Desintegranten ; die Tablette weist, bezogen auf das Gesamtgewicht der Tablette bis zu 5 Gew.-%, des Desintegranten oder Sprengmittels auf; insbesondere ist der Desintegrant ausgewählt aus der Gruppe bestehend aus: Polyvinylpyrrolidon, Polyvinylpolypyrrolidon, Stärke, Stärkederivaten, Carboxymethylcellulose, Salzen und Derivaten von Carboxymethylcellulose und einer Kombination davon.

4. Tablette nach einem der vorhergehenden Ansprüche mit, bezogen auf das Gesamtgewicht der Tablette einem Gewichtsprozentsatz eines oder mehrerer Bindemittel, der niedriger als 10 ist; insbesondere sind das eine oder mehrere Bindemittel ausgewählt aus der Gruppe bestehend aus: Dextrose, Saccharose, Dextrin, vorgelatinierter Stärke, Mannitol und einer Kombination davon.

5. Tablette nach Anspruch 4, bei welcher die Tablette ohne Bindemittel oder Bindemittelfrei ist.

6. Tablette nach einem der vorhergehenden Ansprüche umfassend, bezogen auf das Gesamtgewicht der Tablette bis zu 5 Gew.-% von mindestens einem antiadhäsiven Schmiermittel; insbesondere ist das antiadhäsive Schmiermittel ausgewählt aus der Gruppe bestehend aus: Stärke, Siliziumdioxid, Stearate, Talk, Wachs, Polyethylenglykol, Natrium-/ Magnesiumlaurilsulfat, Leucin und einer Kombination davon.

7. Tablette nach einem der vorhergehenden Ansprüche umfassend, bezogen auf das Gesamtgewicht der Tablette mindestens einen hochintensiven Süßstoff; insbesondere umfasst die Tablette bis zu 30 Gew.-% des hochintensiven Süßstoffs.

8. Tablette nach einem der vorhergehenden Ansprüche umfassend eine funktionelle Komponente; insbesondere umfasst die Tablette, bezogen auf das Gesamtgewicht der Tablette bis zu 40 Gew.-% der funktionellen Komponente; insbesondere ist die funktionelle Komponente ausgewählt aus der Gruppe bestehend aus: Vitaminen, Aminosäuren, Nahrungsmineralien, Antioxidantien, Pflanzenextrakten und einer Kombination davon.

9. Tablette nach einem der vorhergehenden Ansprüche und mit einem Gewicht im Bereich von 0,05 Gramm bis 10 Gramm.

10. Verfahren zur Herstellung einer Tablette, die, bezogen auf das Gesamtgewicht der Tablette mindestens 60 Gew.-% Fructose, die von Früchten, insbesondere von Trauben stammt, umfasst; wobei die Tablette einem der Ansprüche 1 bis 9 entspricht; das Verfahren umfassend: einen Kompressionsschritt, bei dem eine Formulierung, die, bezogen auf das Gesamtgewicht der Formulierung mindestens 60 Gew.-% Fructose, die von Früchten stammt, umfasst, mittels Kompression verarbeitet wird, um die Tablette zu erhalten.

11. Verfahren nach Anspruch 10, umfassend einen Mischschritt, bei dem die aus Früchten (insbesondere aus Trauben) gewonnene Fructose gemischt wird, um die genannte Formulierung zu erhalten; wobei die Formulierung einen Gewichtsprozentsatz von einem oder mehreren Bindemitteln aufweist, der niedriger als 10%, bezogen auf das Gesamtgewicht der Tablette, ist; insbesondere werden das eine oder die mehreren Bindemittel aus der Gruppe ausgewählt, bestehend aus: Dextrose, Saccharose, Dextrin, vorgelatinierter Stärke, Mannitol und einer Kombination davon.

12. Verfahren nach Anspruch 10 oder 11, bei welchem die Mischung ohne Bindemittel oder Bindemittelfrei ist.

13. Verfahren nach einem der Ansprüche 10 bis 12, bei welchem die von Früchten stammende Fructose eine Korngröße im Bereich von 50 µm bis 600 µm aufweist.

14. Tablette nach einem der Ansprüche von 1 bis 9 und erhältlich durch das Verfahren nach einem der Ansprüche von 10 bis 13.

## Revendications

1. Comprimé comprenant au moins 60 % en poids, par rapport au poids total du comprimé, de fructose dérivé de fruit, en particulier de raisins ; dans lequel le fructose est cristallin et présente un δ¹³C (‰) par rapport au V-PDB allant de -30 à -24 et une valeur R supérieure à 2,4 et inférieure à 2,6.

2. Comprimé selon la revendication 1 et comprenant au moins 70 %, en particulier au moins 90 %, en poids, par rapport au poids total du comprimé, de fructose dérivé de fruit.

3. Comprimé selon l'une des revendications précédentes et comprenant au moins un agent de délitement ; le comprimé contient jusqu'à 5 % en poids, par rapport au poids total du comprimé, dudit agent de délitement ; en particulier, ledit agent de délitement est sélectionné dans le groupe consistant en : une polyvinylpyrrolidone, une polyvinylpolypyrrolidone, un amidon, les dérivés d'amidon, une carboxyméthyl cellulose, les sels et les dérivés de carboxyméthyl cellulose et une combinaison de ceux-ci.

4. Comprimé selon l'une des revendications précédentes et présentant un pourcentage en poids d'un ou de plusieurs liants qui est inférieur à 10 %, par rapport au poids total du comprimé ; en particulier, lesdits un ou plusieurs liants sont sélectionnés dans le groupe consistant en : le dextrose, le saccharose, la dextrine, un amidon prégélatinisé, le mannitol et une combinaison de ceux-ci.

5. Comprimé selon la revendication 4, dans lequel le comprimé est sans liant.

6. Comprimé selon l'une quelconque des revendications précédentes et comprenant jusqu'à 5 % poids, par rapport au poids total du comprimé, d'au moins un lubrifiant anti-adhérant ; en particulier, ledit lubrifiant anti-adhérant est sélectionné dans le groupe consistant en : un amidon, une silice, les stéarates, un talc, une cire, un polyéthylène glycol, un lauryl sulfate de sodium/magnésium, une leucine et une combinaison de ceux-ci.

7. Comprimé selon l'une quelconque des revendications précédentes et comprenant au moins un édulcorant à haute intensité ; en particulier, le comprimé comprend jusqu'à 30 % en poids, par rapport au poids total du comprimé, dudit édulcorant à haute intensité.

8. Comprimé selon l'une quelconque des revendications précédentes et comprenant un composant fonctionnel ; en particulier, le comprimé comprend jusqu'à 40 % en poids, par rapport au poids total du comprimé, dudit composant fonctionnel ; en particulier, ledit composant fonctionnel est sélectionné dans le groupe consistant en : les vitamines, les acides aminés, les minéraux alimentaires, les antioxydants, les extraits végétaux et une combinaison de ceux-ci.

9. Comprimé selon l'une quelconque des revendications précédentes et présentant un poids allant de 0,05 gramme à 10 grammes.

10. Méthode de fabrication d'un comprimé comprenant au moins 60 % en poids, par rapport au poids total du comprimé, de fructose dérivé de fruit, en particulier de raisin ; dans laquelle le comprimé est selon l'une quelconque des revendications 1 à 9 ; ladite méthode comprend :
une étape de compression, pendant laquelle une formulation comprenant au moins 60 % en poids, par rapport au poids total de la formulation, de fructose dérivé de fruit est transformée par compression afin d'obtenir le comprimé.

11. Méthode selon la revendication 10, et comprenant une étape de mélange, pendant laquelle le fructose dérivé de fruit (en particulier de raisins) est mélangé afin d'obtenir ladite formulation ; la formulation présentant un pourcentage en poids d'un ou de plusieurs liants qui est inférieur à 10 %, par rapport au poids total du comprimé ; en particulier, lesdits un ou plusieurs liants sont sélectionnés dans le groupe consistant en : le dextrose, le saccharose, la dextrine, un amidon prégélatinisé, le mannitol et une combinaison de ceux-ci.

12. Méthode selon la revendication 10 ou 11, dans laquelle le mélange est sans liant.

13. Méthode selon l'une quelconque des revendications 10 à 12, dans laquelle le fructose dérivé de fruit présente une taille de grain allant de 50 µm à 600 µm.

14. Comprimé selon l'une quelconque des revendications 1 à 9 et pouvant être obtenu par la méthode selon l'une quelconque des revendications 10 à 13.
